# EUROPÄISCHE PATENTSCHRIFT

(11) **EP 3 581 154 B1**
(45) Veröffentlichungstag und Bekanntmachung des Hinweises auf die Patenterteilung: **26.05.2021**
(21) Anmeldenummer: 19166834.2
(22) Anmeldetag: 02.04.2019
(51) Int. Cl.: A61F 2/46

(54) **FILTRIERUNGSVORRICHTUNG FÜR EIN CHIRURGISCHES ABSAUGSYSTEM UND VERFAHREN ZUR ERZEUGUNG EINES MIT AUTOLOGEN GEWEBEBESTANDTEILEN VERSEHENEN KNOCHENERSATZSTOFFES**
FILTERING DEVICE FOR A SURGICAL SUCTION SYSTEM AND METHOD FOR PRODUCING A BONE REPLACEMENT MATERIAL WITH AUTOLOGOUS TISSUE COMPONENTS
DISPOSITIF DE FILTRATION POUR UN SYSTÈME D'ASPIRATION CHIRURGICALE ET PROCÉDÉ DE PRODUCTION D'UN SUBSTITUT OSSEUX POURVU DE COMPOSANTS DE TISSU AUTOLOGUES

(30) Priorität: 14.06.2018 DE 102018114304
(43) Veröffentlichungstag der Anmeldung: 18.12.2019
(73) Patentinhaber: Jäger, Marcus, 45133 Essen (DE)
(72) Erfinder: Jäger, Marcus, 45133 Essen (DE)
(74) Vertreter: Andrejewski - Honke Patent- und Rechtsanwälte Partnerschaft mbB

(56) Entgegenhaltungen:
- DE-U1- 29 621 636
- US-A1- 2008 071 192
- US-A1- 2016 324 530

## Beschreibung

Die Erfindung betrifft eine Filtrierungsvorrichtung für ein chirurgisches Absaugsystem mit einem Einlassabschnitt, einem Auslassabschnitt und einer Filterkammer, wobei die Filterkammer zwischen dem Einlassabschnitt und dem Auslassabschnitt angeordnet ist und wobei in der Filterkammer zumindest ein Filterelement angeordnet ist. Fernerhin betrifft die Erfindung ein Verfahren zur Erzeugung eines mit autologen Gewebebestandteilen versehenen Knochenersatzstoffes, ein Filterelement, sowie die Verwendung eines derartigen Knochenersatzstoffes bzw. Filterelementes in einem Verfahren zur Behandlung von Knochendefekten im menschlichen Körper.

Filtrierungsvorrichtungen der eingangs genannten Art sind aus der Praxis in unterschiedlichen Ausführungsformen grundsätzlich bekannt. Solche Filtrierungsvorrichtungen kommen in chirurgischen Absaugsystemen zum Einsatz, die im Zuge von Operationen, insbesondere am menschlichen Körper, beispielsweise Operationen am Knochen, verwendet werden. Die chirurgischen Absaugsysteme dienen dazu, Blut und freigesetzte lose Gewebebestandteile, wie Knochenpartikel, abzusaugen. Derartige Absaugsysteme weisen dazu in der Regel eine Absaugvorrichtung in Form einer Pumpe auf und ihnen sind zumeist weitere Geräte bzw. Folgesysteme wie Autotransfusionssysteme nachgeschaltet. Solche Folgesysteme würden durch abgesaugte Gewebebestandteile, beispielsweise durch Knochenreste, in ihrer Funktionsfähigkeit beeinträchtigt. Aus diesem Grund werden in den chirurgischen Absaugsystemen oftmals Filtrierungsvorrichtungen bzw. Filterelemente eingesetzt, um derartige Bestandteile zurückzuhalten.

Die aus der Praxis bekannten Filtrierungsvorrichtungen bzw. Filterelemente weisen allerdings eine Reihe von Nachteilen auf. Diese Filtrierungsvorrichtungen bzw. Filterelemente werden in der Regel durch die abgesaugten Gewebebestandteile schnell zugesetzt, sodass sie verstopfen und ausgetauscht werden müssen. Dabei werden zumeist Einweg-Filtrierungsvorrichtungen verwendet, in denen das Filterelement unzugänglich und fest in einem Gehäuse fixiert bzw. platziert ist. Im Falle eines zugesetzten bzw. verstopften Filterelementes wird sowohl das Filterelement, als auch das Gehäuse entsorgt. Dies ist nur wenig wirtschaftlich. Fernerhin werden die mit den bekannten Filtrierungsvorrichtungen bzw. Filterelementen aufgefangene bzw. zurückgehaltene Gewebebestandteile ungenutzt zusammen mit der Filtrierungsvorrichtung bzw. mit dem Filterelement entsorgt. Dies ist sowohl in wirtschaftlicher, als auch in medizinischer Hinsicht unvorteilhaft. Die von den Filterelementen aufgefangenen bzw. zurückgehaltenen Gewebebestandteile besitzen ein osteoregeneratives Potenzial. Sie sind somit zur Knochenneubildung bzw. zur Behandlung von Knochendefekten geeignet. Dieses osteoregenerative Potenzial wird bei den aus der Praxis bekannten Maßnahmen nicht ausgenutzt. Bei vielen der bekannten Systeme ist es überhaupt nicht möglich, die Gewebebestandteile aus der Filtrierungsvorrichtung bzw. aus dem Filterelement zu isolieren bzw. wäre eine solche Maßnahme mit einem erheblichen Aufwand verbunden. - Insoweit besteht Verbesserungsbedarf.

Aus US 2016/324530 A1 ist ein Verfahren zur Erzeugung eines Knochenersatzstoffes bekannt. Hier wird eine Filtrierungsvorrichtung für ein chirurgisches Absaugsystem verwendet. Die Filtrierungsvorrichtung weist einen Einlassabschnitt für ein Fluid auf, das autologe Gewebebestandteile enthält. Das Fluid durchströmt die Filterkammer und somit ein Filterelement und anschließend strömt das filtrierte Fluid durch einen Auslassabschnitt aus der Filtrierungsvorrichtung heraus. Diese Maßnahmen weisen den Nachteil auf, dass autologe Gewebebestandteile nicht effizient weiterverwendet werden können bzw. nur mit relativ großem Aufwand weiterverwendet werden können.

Der Erfindung liegt das technische Problem zugrunde, eine Filtrierungsvorrichtung der eingangs genannten Art anzugeben, mit der die vorstehend beschriebenen Nachteile effektiv und auf einfache Art und Weise vermieden werden können, die insbesondere über einen ausreichend langen Zeitraum funktionssicher arbeitet, die in wirtschaftlicher Hinsicht allen Anforderungen genügt und die zudem dazu geeignet ist, das osteoregenerative Potenzial der abgesaugten Gewebebestandteile auszunutzen bzw. nutzbar zu machen. Fernerhin liegt der Erfindung das technische Problem zugrunde, ein Verfahren zur Erzeugung eines mit autologen Gewebebestandteilen versehenen Knochenersatzstoffes, ein Filterelement, sowie eine Verwendung eines solchen Knochenersatzstoffes bzw. Filterelementes in einem Verfahren zur Behandlung von Knochendefekten im menschlichen Körper anzugeben.

Zur Lösung des technischen Problems lehrt die Erfindung eine Filtrierungsvorrichtung für ein chirurgisches Absaugsystem, wobei die Filtrierungsvorrichtung einen Einlassabschnitt, einen Auslassabschnitt und eine Filterkammer aufweist, wobei die Filterkammer zwischen dem Einlassabschnitt und dem Auslassabschnitt angeordnet ist, wobei in der Filterkammer zumindest ein Filterelement angeordnet ist, wobei
entweder das Filterelement zumindest bereichsweise auf Basis zumindest eines Knochenersatzstoffes, vorzugsweise eines bioresorbierbaren Knochenersatzstoffes, ausgebildet ist
oder das Filterelement zumindest bereichsweise auf Basis zumindest eines Trägermaterials - insbesondere auf Basis zumindest eines Kunststoffes - ausgebildet ist, wobei innerhalb des Filterelementes zumindest ein Knochenersatzstoff - vorzugsweise zumindest ein bioresorbierbarer Knochenersatzstoff - angeordnet ist
und wobei mittels des Filterelementes autologe Gewebebestandteile, die in einem die Filtrierungsvorrichtung durchströmenden Fluid mitgeführt werden, abtrennbar sind.
- Zweckmäßigerweise ist bei dieser zweiten Ausführungsform der Erfindung das Trägermaterial bzw. der Kunststoff ein polymerer Kunststoff, vorzugsweise ein polymeres Acrylat oder ein polymeres Methacrylat. Besonders bevorzugt ist das Trägermaterial für das Filterelement Polymethylmethacrylat (PMMA). Es liegt im Rahmen der Erfindung, dass bei einem als Hohlkörper ausgebildeten Filterelement die Wandungen aus dem Trägermaterial bzw. aus dem vorgenannten Kunststoff bestehen bzw. im Wesentlichen bestehen. Bei dieser zweiten erfindungsgemäßen Ausführungsform ist der in dem Filterelement aufgenommene Knochenersatzstoff - insbesondere bioresorbierbarer Knochenersatzstoff - vorzugsweise in Form von Teilchen bzw. kleinen Teilchen und insbesondere in Form von Granulatteilchen ausgebildet und innerhalb des Filterelementes angeordnet. Wenn das Filterelement - wie weiter unten erläutert - Fluiddurchtrittsöffnungen aufweist, liegt es fernerhin im Rahmen der Erfindung, dass der Durchmesser der Teilchen bzw. Granulatteilchen kleiner ist als der Durchmesser der Fluiddurchtrittsöffnungen. Auf diese Weise wird verhindert, dass die Teilchen aus dem Filterelement gelangen und dass diese Teilchen bei einem Saugvorgang effektiv mit den autologen Gewebebestandteilen benetzt werden. Grundsätzlich können die Teilchen des Knochenersatzstoffes in Form von Chips, kleinen Kügelchen, Würfeln, Quadern oder dergleichen ausgebildet sein.

Es liegt im Rahmen der Erfindung, dass bei der ersten Ausführungsform, bei der das Filterelement zumindest bereichsweise auf Basis zumindest eines Knochenersatzstoffes ausgebildet ist, dieses Filterelement zusätzlich mit einem Knochenersatzstoff - insbesondere in Form von Teilchen bzw. kleinen Teilchen - gefüllt ist.

Chirurgisches Absaugsystem meint im Rahmen der Erfindung insbesondere ein im Zuge von Operationen - vorzugsweise am menschlichen Körper - verwendetes Absaugsystem, mit dem ein Fluid, das aus Fett, Knochenmark, Blut und anderen Gewebebestandteilen besteht, abgesaugt wird. Dieses Fluid enthält auch feste Gewebebestandteile wie Knorpelreste und Knochenreste und außerdem hochviskose Bestandteile wie Blutgerinnsel, Fette und dergleichen. Ein solches chirurgisches Absaugsystem kann eine Absaugvorrichtung in Form einer Pumpe und fernerhin eine Absaugeinrichtung, beispielsweise einen Absaugrüssel bzw. ein Absaugrohr oder dergleichen aufweisen, mit dem das Fluid an der Operationsstelle abgesaugt wird. Es kann zudem zumindest ein Auffangbehälter für das Fluid vorgesehen sein. Zwischen der Absaugeinrichtung und dem Auffangbehälter für das Fluid ist im Rahmen der Erfindung eine erfindungsgemäße Filtrierungsvorrichtung angeordnet mit der die festen und/oder hochviskosen Gewebebestandteile aus dem abgesaugten Fluid abtrennbar sind.

Mit dem Ausdruck Fluid bzw. die Filtrierungsvorrichtung durchströmendes Fluid ist im Rahmen der Erfindung also insbesondere das mit dem chirurgischen Absaugsystem im Zuge einer Operation abgesaugte Fluid gemeint.

Autologe Gewebebestandteile meint im Rahmen der Erfindung insbesondere körpereigene Gewebebestandteile, die Teil des mittels des chirurgischen Absaugsystems abgesaugten Fluides sind bzw. die in diesem Fluid mitgeführt werden.

Der Ausdruck Knochenersatzstoff bezeichnet im Rahmen der Erfindung insbesondere einen Stoff, der zur Auffüllung von Knochendefekten - insbesondere am bzw. im menschlichen Knochen - verwendet werden kann, wenn eigenes Knochenmaterial nicht oder nicht in ausreichendem Maße zur Verfügung steht. Bioresorbierbarer Knochenersatzstoff meint in diesem Zusammenhang insbesondere einen solchen Knochenersatzstoff, der sich im Körper, vorzugsweise im menschlichen Körper, nach einer gewissen Zeit selbst abbaut bzw. auflöst oder durch Zellen und/oder Gewebeflüssigkeiten abgebaut wird. Solche bioresorbierbaren Knochenersatzstoffe werden demzufolge auch als biodegradierbare Knochenersatzstoffe oder selbstauflösende Knochenersatzstoffe bezeichnet.

Filterelement meint im Rahmen der Erfindung insbesondere ein Element, durch das ein Fluid hindurchtreten kann, während in dem Fluid mitgeführte Bestandteile, beispielsweise Feststoffe oder hochviskose Stoffe von dem Filterelement - zumindest teilweise - aufgefangen bzw. zurückgehalten werden.

Eine besonders bevorzugte Ausführungsform der erfindungsgemäßen Filtrierungsvorrichtung ist dadurch gekennzeichnet, dass das Filterelement als Hohlkörper ausgebildet ist. Vorzugsweise weist das als Hohlkörper ausgebildete Filterelement einen inneren Strömungsraum auf und ganz besonders bevorzugt umgibt die Filterkammer der Filtrierungsvorrichtung das Filterelement. Zweckmäßigerweise umgibt die Filterkammer das Filterelement derart, dass zwischen der Außenseite des Filterelementes und der Innenseite der Filterkammer ein äußerer Strömungsraum angeordnet ist. Strömungsraum meint in diesem Zusammenhang insbesondere einen Raum, in dem das Fluid strömen kann bzw. den das Fluid durchströmen kann.

Erfindungsgemäß weist die Filtrierungsvorrichtung zumindest einen Einlassabschnitt, zumindest einen Auslassabschnitt und zumindest eine Filterkammer auf und in der Filterkammer ist das zumindest eine Filterelement angeordnet. Es empfiehlt sich, dass der innere Strömungsraum des Filterelementes in Fluidverbindung mit dem Einlassabschnitt steht. Der Einlassabschnitt der Filtrierungsvorrichtung dient dazu, das mit dem chirurgischen Absaugsystem abgesaugte Fluid in die Filterkammer einzulassen bzw. einzuführen. Wenn gemäß bevorzugter Ausführungsform der innere Strömungsraum in Fluidverbindung mit dem Einlassabschnitt steht, dann gelangt das Fluid vorzugsweise durch den Einlassabschnitt in den inneren Strömungsraum des Filterelementes.

Es hat sich bewährt, dass der äußere Strömungsraum in Fluidverbindung mit dem Auslassabschnitt steht. Der erfindungsgemäße Auslassabschnitt dient dazu, das Fluid aus der Filterkammer herauszuführen bzw. herauszulassen. Zweckmäßigerweise muss das die Filtrierungsvorrichtung durchströmende Fluid das Filterelement durchtreten, um von dem inneren Strömungsraum in den äußeren Strömungsraum zu gelangen. Von dem äußeren Strömungsraum gelangt das filtrierte Fluid bzw. Filtrat dann vorzugsweise über den Auslassabschnitt aus der Filterkammer bzw. aus der Filtrierungsvorrichtung heraus.

Es liegt im Rahmen der Erfindung, dass der Einlassabschnitt filterkammerseitig ausschließlich in Fluidverbindung mit dem inneren Strömungsraum des Filterelementes steht und/oder dass der Auslassabschnitt filterkammerseitig ausschließlich in Fluidverbindung mit dem äußeren Strömungsraum steht. Dieser Ausführungsform liegt die Erkenntnis zugrunde, dass das Fluid dann durch den Einlassabschnitt in die Filterkammer gelangt und direkt in den inneren Strömungsraum des Filterelementes geführt wird. Bevorzugt muss anschließend das Fluid das als Hohlkörper ausgebildete Filterelement - von innen nach außen - durchtreten und gelangt auf diese Weise in den äußeren Strömungsraum. Wenn der Auslassabschnitt filterkammerseitig zweckmäßigerweise ausschließlich in Fluidverbindung mit dem äußeren Strömungsraum steht, wird das filtrierte Fluid dann anschließend durch den Auslassabschnitt aus der Filterkammer bzw. aus der Filtrierungsvorrichtung herausgeführt. Mit dem Begriff ausschließliche Fluidverbindung ist im Rahmen der Erfindung im Übrigen die direkte Fluidverbindung gemeint, die nicht den Durchtritt durch das Filterelement betrifft bzw. ausschließt. Grundsätzlich liegt es auch im Rahmen der Erfindung, dass der innere Strömungsraum in Fluidverbindung mit dem Auslassabschnitt steht bzw. dass der Auslassabschnitt filterkammerseitig ausschließlich in Fluidverbindung mit dem inneren Strömungsraum steht und/oder dass der äußere Strömungsraum in Fluidverbindung mit dem Einlassabschnitt steht bzw. dass der Einlassabschnitt filterkammerseitig ausschließlich in Fluidverbindung mit dem äußeren Strömungsraum steht. Im Rahmen einer solchen Ausführungsform ist die Filtrierungsvorrichtung dann dafür vorgesehen, dass das abgesaugte Fluid zunächst durch den Einlassabschnitt in den äußeren Strömungsraum gelangt, anschließend das als Hohlkörper ausgebildete Filterelement - von außen nach innen - durchtritt und von dem inneren Strömungsraum dann über den Auslassabschnitt aus der Filtrierungsvorrichtung herausgelassen bzw. herausgeführt wird.

Gemäß einer besonders bevorzugten Ausführungsform der Erfindung ist das Filterelement röhrenförmig ausgebildet. Ein solches röhrenförmiges Filterelement weist vorzugsweise an seinen Stirnseiten jeweils eine Öffnung auf und zweckmäßigerweise besitzt das röhrenförmige Filterelement eine sechseckige bzw. hexagonale Querschnittsfläche. Gemäß einer alternativen Ausführungsvariante ist die Querschnittsfläche des Filterelementes kreisförmig ausgebildet. Grundsätzlich liegen auch andere Querschnittsflächengeometrien des röhrenförmigen Filterelementes, beispielsweise quadratische, rechteckige, dreieckige, sternförmige, ovale und dergleichen im Rahmen der Erfindung.

Wenn das Filterelement gemäß bevorzugter Ausführungsform röhrenförmig ausgebildet ist, empfiehlt es sich, dass im montierten Zustand des Filterelementes zumindest ein Ende des Filterelementes verschlossen ist. Besonders bevorzugt ist im montierten Zustand des Filterelementes das dem Auslassabschnitt zugewandte bzw. zugeordnete Ende bzw. die dem Auslassabschnitt zugewandte bzw. zugeordnete Öffnung des Filterelementes verschlossen. Dieser Ausführungsform liegt die Erkenntnis zugrunde, dass dann das Fluid vorzugsweise über den Einlassabschnitt und die offene Stirnseite bzw. das offene Ende des röhrenförmigen Filterelementes in den inneren Strömungsraum gelangen kann und anschließend nicht an der gegenüberliegenden Stirnseite des Filterelementes aus dem Filterelement austreten kann, sondern insbesondere das Filterelement durchtreten muss, um in den äußeren Strömungsraum zu gelangen. Montierter Zustand des Filterelementes meint in diesem Zusammenhang insbesondere denjenigen Zustand des Filterelementes, in dem es sich in der Filterkammer befindet und in dem die Filtrierungsvorrichtung einsatzbereit ist.

Der Verschluss eines Endes des röhrenförmigen Filterelementes kann bevorzugt durch ein an der Filtrierungsvorrichtung angeordnetes Verschlusselement, beispielsweise durch eine Verschlussplatte oder dergleichen erfolgen. Ein solches Verschlusselement bzw. eine solche Verschlussplatte kann besonders bevorzugt an dem Auslassabschnitt oder alternativ an bzw. in der Filterkammer - empfohlenermaßen an der dem Auslassabschnitt zugewandten bzw. zugeordneten Seite der Filterkammer - vorgesehen sein. Zweckmäßigerweise ist das Verschlusselement als Verschlussplatte ausgebildet. Das Verschlusselement bzw. die Verschlussplatte weist dann zweckmäßigerweise einen Befestigungsfortsatz auf, auf den das Filterelement aufgesteckt bzw. aufgeschoben werden kann bzw. der in das Filterelement eingesteckt bzw. eingeschoben werden kann, sodass der Befestigungsfortsatz im montierten Zustand des Filterelementes in das Filterelement hineinreicht. Empfohlenermaßen ist außerdem an dem Einlassabschnitt ein Manschettenelement mit einem Befestigungsfortsatz vorgesehen, wobei vorzugsweise das Filterelement auf den Befestigungsfortsatz aufgesteckt bzw. aufgeschoben werden kann, sodass der Befestigungsfortsatz im montierten Zustand des Filterelementes in das Filterelement hinreicht. Dann wird das Filterelement ganz besonders bevorzugt von dem bzw. zwischen dem Befestigungsfortsatz des Verschlusselementes bzw. der Verschlussplatte und dem Befestigungsfortsatz des Manschettenelementes in der Filterkammer gehalten. Grundsätzlich liegt es auch im Rahmen der Erfindung, dass das Verschlusselement bzw. die Verschlussplatte an dem Filterelement selbst angeordnet ist bzw. Teil des Filterelementes ist.

Eine Ausführungsform, der im Rahmen der Erfindung ganz besondere Bedeutung zukommt, ist dadurch gekennzeichnet, dass die Filterkammer röhrenförmig ausgebildet ist. Ganz besonders bevorzugt sind sowohl die Filterkammer als auch das Filterelement röhrenförmig ausgebildet und zweckmäßigerweise umgibt die röhrenförmige Filterkammer das röhrenförmige Filterelement. Zweckmäßigerweise weist die Filterkammer eine kreisförmige Querschnittsfläche auf. Grundsätzlich liegen auch andere Querschnittsflächengeometrien der Filterkammer wie hexagonale bzw. sechseckige, quadratische, rechteckige und dergleichen im Rahmen der Erfindung.

Es liegt fernerhin im Rahmen der Erfindung, dass das Filterelement und die Filterkammer koaxial zueinander angeordnet sind. Zweckmäßigerweise erstreckt sich das Filterelement in seiner Längsausdehnung über einen Großteil der Länge der Filterkammer, vorzugsweise über zumindest 70 % der Länge der Filterkammer, besonders bevorzugt über zumindest 80 % der Länge der Filterkammer und ganz besonders bevorzugt über zumindest 90 % der Länge der Filterkammer, beispielsweise über zumindest 95 % der Länge der Filterkammer. Gemäß ganz besonders bevorzugter Ausführungsform erstreckt sich das Filterelement in seiner Längsausdehnung über die gesamte Länge der Filterkammer bzw. im Wesentlichen über die gesamte Länge der Filterkammer.

Eine sehr empfohlene Ausführungsform der Erfindung ist dadurch gekennzeichnet, dass der Einlassabschnitt und/oder der Auslassabschnitt an der der Filterkammer abgewandten Seite zumindest ein Anschlusselement, vorzugsweise einen Anschlussstutzen, aufweist. Mittels eines solchen Anschlusselementes kann die erfindungsgemäße Filtrierungsvorrichtung mit weiteren Einrichtungen des chirurgischen Absaugsystems verbunden werden. Insbesondere sind die Anschlusselemente dazu geeignet, dass Schläuche oder Rohre oder dergleichen mit der Filtrierungsvorrichtung verbunden werden können. Dazu kann das Anschlusselement beispielsweise als konisch zulaufender Anschlussstutzen mit einem Fluiddurchlass - beispielsweise in Form eines Innenrohres - ausgebildet sein, der in Richtung der von der Filterkammer abgewandten Seite konisch zuläuft. Grundsätzlich liegen aber auch andere Anschlusselemente, die an der der Filterkammer abgewandten Seite des Einlassabschnittes und/oder des Auslassabschnittes angeordnet sind im Rahmen der Erfindung. Gemäß einer Ausführungsform der Erfindung weist der Einlassabschnitt ein Anschlusselement auf, in das eine Absaugeinrichtung, beispielsweise ein Absaugrohr oder ein Absaugrüssel, eingeführt bzw. eingesteckt werden kann, und mittels der Absaugeinrichtung wird das Fluid abgesaugt bzw. angesaugt. Wenn gemäß bevorzugter Ausführungsform der Erfindung ein Manschettenelement mit einem Befestigungsfortsatz an dem Einlassabschnitt vorgesehen ist, liegt es im Rahmen der Erfindung, dass ein Fluiddurchlass des Einlassabschnittes - beispielsweise in Form eines Innenrohres - das Manschettenelement durchgreift und das vorzugsweise eine Absaugeinrichtung, beispielsweise ein Absaugrohr oder ein Absaugrüssel, in das Innenrohr des Einlassabschnittes eingeführt bzw. eingesteckt werden kann und dass die Absaugeinrichtung dann bevorzugt das Innenrohr und/oder das Manschettenelement und den Befestigungsfortsatz durchgreift. Grundsätzlich liegt es auch im Rahmen der Erfindung, dass der Einlassabschnitt der Filtrierungsvorrichtung selbst direkt zum Absaugen bzw. Ansaugen eingesetzt wird. Vorzugsweise dient das Anschlusselement des Auslassabschnittes dazu, dass der Auslassabschnitt mit einem Schlauch verbunden wird, über den das filtrierte Fluid dann weitergeführt wird, beispielsweise in einen Auffangbehälter oder dergleichen.

Nach einer bewährten Ausführungsform der Erfindung ist an dem Einlassabschnitt und/oder an dem Auslassabschnitt filterkammerseitig zumindest ein Verbindungselement, vorzugsweise ein Gewinde vorgesehen. Das Gewinde ist besonders bevorzugt als Innengewinde ausgebildet. Es empfiehlt sich, dass das an dem Einlassabschnitt und/oder an dem Auslassabschnitt filterkammerseitig angeordnete Verbindungselement mit einem an der Filterkammer angeordneten komplementären Verbindungselement, vorzugsweise mit einem Komplementärgewinde, wechselwirkt. Das Komplementärgewinde ist besonders bevorzugt als Komplementäraußengewinde ausgebildet. Es hat sich bewährt, dass auf diese Weise der Einlassabschnitt und/oder der Auslassabschnitt reversibel von der Filterkammer entfernbar - vorzugsweise abschraubbar - ist. Grundsätzlich liegt es auch im Rahmen der Erfindung, dass an dem Einlassabschnitt und/oder an dem Auslassabschnitt ein Außengewinde vorgesehen ist, das mit einem an der Filterkammer angeordneten Komplementärinnengewinde wechselwirkt. Es liegt fernerhin im Rahmen der Erfindung, dass das Verbindungselement und das komplementäre Verbindungselement als Steckverbindungen ausgebildet sind. Durch die reversible Entfernbarkeit des Einlassabschnittes und/oder des Auslassabschnittes von der Filterkammer kann erreicht werden, dass die Filtrierungsvorrichtung einfach und funktionssicher geöffnet werden kann und dass das Filterelement dann aus der Filtrierungsvorrichtung entnommen werden kann. Das Filterelement kann somit problemlos entfernt und weiterverwendet werden. Zudem liegt dieser Ausführungsform die Erkenntnis zugrunde, dass ein neues, unbenutztes Filterelement problemlos in die Filtrierungsvorrichtung eingebracht werden kann und somit kann die Filterkammer und/oder der Einlassabschnitt und/oder der Auslassabschnitt wiederverwendet werden. An einem reversibel von der Filterkammer entfernbaren Einlassabschnitt und/oder Auslassabschnitt kann im Übrigen auch das obenstehend bereits erwähnte Verschlusselement, insbesondere eine Verschlussplatte, angeordnet sein, die zum Verschluss eines röhrenförmigen Filterelementes an einer Stirnseite des Filterelementes dient. Grundsätzlich können sowohl der Einlassabschnitt, als auch der Auslassabschnitt filterkammerseitig mit zumindest einem Verbindungselement versehen sein und dann ist die Filterkammer an ihrer dem Einlassabschnitt und an ihrer dem Auslassabschnitt zugeordneten Seite jeweils mit komplementären Verbindungselementen versehen. Gemäß einer alternativen und besonders bevorzugten Ausführungsform ist entweder der Einlassabschnitt oder der Auslassabschnitt mit einem Verbindungselement versehen und reversibel von der Filterkammer entfernbar. Dann liegt es im Rahmen der Erfindung, dass der jeweils andere Abschnitt einstückig mit der Filterkammer ausgebildet ist und gleichsam nicht reversibel von der Filterkammer entfernbar ist.

Nach besonders empfohlener Ausführungsform der Erfindung beträgt die Stärke bzw. Wandstärke s des Filterelementes zwischen 0,5 mm und 4,0 mm, bevorzugt zwischen 0,7 mm und 3,0 mm, besonders bevorzugt zwischen 1,0 mm und 2,5 mm und ganz besonders bevorzugt zwischen 1,2 mm und 2,0 mm. Stärke bzw. Wandstärke s des Filterelementes meint im Rahmen der Erfindung insbesondere die Dicke bzw. die Wanddicke des Filterelementes, die das Fluid durchtreten muss, um das Filterelement zu durchtreten. Insoweit meint Stärke bzw. Wandstärke s des Filterelementes besonders bevorzugt die Durchtrittsdicke des Filterelementes. Im Falle eines Filterelementes, das als Hohlkörper ausgebildet ist, beispielsweise röhrenförmig ausgebildet ist, meint Stärke bzw. Wandstärke s die Wanddicke des röhrenförmigen Filterelementes. Wenn das Filterelement gemäß der ersten Ausführungsform der Erfindung zumindest bereichsweise auf Basis zumindest eines Knochenersatzstoffes ausgebildet ist, kann das Filterelement auch als einfache Filterscheibe oder dergleichen ausgebildet sein und dann meint die Stärke bzw. Wandstärke s des Filterelementes insbesondere die Dicke bzw. Durchtrittsdicke eines solchen scheibenförmigen Filterelementes.

Eine ganz besonders bevorzugte Ausführungsform der Erfindung ist dadurch gekennzeichnet, dass das Filterelement eine Vielzahl von Fluiddurchtrittsöffnungen aufweist. Derartige Fluiddurchtrittsöffnungen sind insbesondere als Fluiddurchtrittslöcher in dem Filterelement vorgesehen. Besonders bevorzugt tritt das Fluid lediglich bzw. im Wesentlichen lediglich durch die Fluiddurchtrittsöffnungen hindurch und passiert auf diese Weise das Filterelement bzw. tritt auf diese Weise durch das Filterelement hindurch. Durch die Wahl der Größe bzw. des Durchmessers der Fluiddurchtrittsöffnungen kann dann bestimmt werden, welche Bestandteile des Fluides aufgrund ihrer Größe die Fluiddurchtrittsöffnungen nicht durchtreten können und gleichsam von dem Filterelement aufgefangen bzw. zurückgehalten werden. Es liegt im Rahmen der Erfindung, dass die Fluiddurchtrittsöffnungen einen Durchmesser a im Bereich von 0,1 mm bis 8,0 mm, bevorzugt zwischen 0,2 mm und 6,0 mm, besonders bevorzugt zwischen 0,5 mm und 4,0 mm und ganz besonders bevorzugt zwischen 1,0 mm und 3,0 mm aufweisen. Durchmesser a meint in diesem Zusammenhang insbesondere den größten Durchmesser einer Fluiddurchtrittsöffnung. Vorzugsweise sind die Fluiddurchtrittsöffnungen als Rundlöcher vorgesehen. Im Falles eines gemäß bevorzugter Ausführungsform röhrenförmig ausgebildeten Filterelementes sind die Fluiddurchtrittsöffnungen besonders bevorzugt in den Wandungen des röhrenförmigen Filterelementes angeordnet und ganz besonders bevorzugt als Rundlöcher ausgebildet. Grundsätzlich liegen aber auch andere Formen von Fluiddurchtrittsöffnungen im Rahmen der Erfindung, beispielsweise Fluiddurchtrittsöffnungen mit eckiger Gestalt, etwa quadratische oder rechteckige Fluiddurchtrittsöffnungen.

Es empfiehlt sich, dass der Flächenanteil der Fluiddurchtrittsöffnungen in Bezug auf die Oberfläche des Filterelementes zwischen 0,5 % und 25 %, bevorzugt zwischen 2 % und 20 %, besonders bevorzugt zwischen 3 % und 15 % und ganz besonders bevorzugt zwischen 4 % und 12 % beträgt. Flächenanteil der Fluiddurchtrittsöffnungen in Bezug auf die Oberfläche des Filterelementes meint den Anteil der summierten Öffnungsfläche der Fluiddurchtrittsöffnungen in Bezug auf die gesamte Oberfläche des Filterelementes ohne solche Öffnungen. Vorzugsweise sind die Fluiddurchtrittsöffnungen in Längsrichtung des Filterelementes gleichmäßig bzw. im Wesentlichen gleichmäßig entlang des Filterelementes bzw. der Oberfläche des Filterelementes angeordnet. Es empfiehlt sich, dass die Fluiddurchtrittsöffnungen in Längsrichtung des Filterelementes mit konstantem Abstand bzw. im Wesentlichen mit konstantem Abstand zueinander angeordnet sind.

Nach der ersten erfindungsgemäßen Ausführungsform ist das Filterelement zumindest bereichsweise auf Basis zumindest eines Knochenersatzstoffes ausgebildet. Vorzugsweise ist das Filterelement vollständig auf Basis des zumindest einen Knochenersatzstoffes ausgebildet. Gemäß sehr empfohlener Ausführungsform der Erfindung besteht das Filterelement aus dem zumindest einen Knochenersatzstoff bzw. besteht das Filterelement im Wesentlichen aus dem zumindest einen Knochenersatzstoff. Dieser Ausführungsform liegt die Erkenntnis zugrunde, dass das Filterelement dann vollständig bzw. im Wesentlichen vollständig aus zumindest einem Knochenersatzstoff, insbesondere aus zumindest einem bioresorbierbaren Knochenersatzstoff bestehen kann und dann eignet sich das nach der Filtration mit autologen Gewebebestandteilen versehene Filterelement unmittelbar dazu, Knochendefekte im menschlichen Körper zu behandeln, da das Filterelement aus keinen weiteren Bestandteilen besteht, als aus dem zumindest einen bioresorbierbaren Knochenersatzstoff.

Eine Ausführungsform, der im Rahmen der Erfindung ganz besondere Bedeutung zukommt, ist dadurch gekennzeichnet, dass es sich bei dem zumindest einen Knochenersatzstoff um einen bioresorbierbaren Knochenersatzstoff handelt. Vorzugsweise handelt es sich bei dem Knochenersatzstoff um ein Calciumsalz einer Säure ausgewählt aus der Gruppe: "Phosphorsäure, Schwefelsäure, Kohlensäure". Besonders bevorzugt handelt es sich bei dem Knochenersatzstoff um ein Calciumphosphat und ganz besonders bevorzugt um β-Tricalciumphosphat. Weiterhin kann auch Hydroxylapatit als Knochenersatzstoff eingesetzt werden. Gemäß einer alternativen Ausführungsvariante der Erfindung handelt es sich bei dem Knochenersatzstoff um ein Polylactid bzw. um ein Bioglas.

Es liegt im Rahmen der Erfindung, dass die Filterkammer und/oder der Einlassabschnitt und/oder der Auslassabschnitt aus zumindest einem polymeren Kunststoff, vorzugsweise aus Polymethylmethacrylat (PMMA) besteht bzw. im Wesentlichen besteht. Es liegt auch im Rahmen der Erfindung, dass die Filterkammer und/oder der Einlassabschnitt und/oder Auslassabschnitt aus zumindest einem polymeren Kunststoff ausgewählt aus der Gruppe: "Polypropylen, Polyethylen, Polyester, Polystyrol, Polymethylmethacrylat, Polycarbonat" ausgewählt ist. Vorzugsweise besteht das gesamte Aggregat aus Filterkammer, Einlassabschnitt und Auslassabschnitt aus Polymethylmethacrylat (PMMA) bzw. im Wesentlichen aus Polymethylmethacrylat (PMMA).

Gegenstand der Erfindung ist weiterhin ein Verfahren zur Erzeugung eines mit autologen Gewebebestandteilen versehenen Knochenersatzstoffes, wobei in eine Filtrierungsvorrichtung für ein chirurgisches Absaugsystem über einen Einlassabschnitt ein Fluid, das autologe Gewebebestandteile enthält, eingesaugt wird, wobei das Fluid anschließend in eine Filterkammer strömt, in der zumindest ein Filterelement angeordnet ist, wobei
entweder das Filterelement zumindest bereichsweise auf Basis zumindest eines Knochenersatzstoffes, vorzugsweise eines bioresorbierbaren Knochenersatzstoffes, ausgebildet ist,
oder das Filterelement zumindest bereichsweise auf Basis zumindest eines Trägermaterials - insbesondere auf Basis zumindest eines Kunststoffes - ausgebildet ist, wobei innerhalb des Filterelementes zumindest ein Knochenersatzstoff - vorzugsweise ein bioresorbierbarer Knochenersatzstoff angeordnet ist,
wobei das Fluid das Filterelement durchtritt, wobei zumindest ein Teil der autologen Gewebebestandteile von dem Filterelement aufgefangen bzw. zurückgehalten wird und wobei das filtrierte Fluid anschließend durch einen Auslassabschnitt aus der Filtrierungsvorrichtung herausströmt.

Die Erfindung betrifft fernerhin nach einer zweiten Ausführungsform ein Verfahren zur Erzeugung eines mit autologen Gewebebestandteilen versehenen Knochenersatzstoffes, wobei in eine Filtrierungsvorrichtung für ein chirurgisches Absaugsystem - insbesondere eine Filtrierungsvorrichtung der oben beschriebenen Art - über einen Einlassabschnitt ein Fluid, das autologe Gewebebestandteile enthält, eingesaugt wird, wobei das Fluid anschließend in eine Filterkammer strömt, in der zumindest ein Filterelement angeordnet ist, wobei das Filterelement zumindest bereichsweise auf Basis zumindest eines Trägermaterials - insbesondere auf Basis zumindest eines Kunststoffes - ausgebildet ist, wobei innerhalb des Filterelementes zumindest ein Knochenersatzstoff - insbesondere ein bioresorbierbarer Knochenersatzstoff - angeordnet ist, wobei das Fluid das Filterelement durchtritt, wobei zumindest ein Teil der autologen Gewebebestandteile von dem Filterelement bzw. von dem in den Filterelement angeordneten Knochenersatzstoff aufgefangen bzw. zurückgehalten wird und wobei das filtrierte Fluid anschließend durch einen Auslassabschnitt aus der Filtrierungsvorrichtung herausströmt.

Es liegt im Rahmen der Erfindung, dass das mit den autologen Gewebebestandteilen versehene Filterelement bzw. das mit den autologen Gewebebestandteilen und dem Knochenersatzstoff versehene Filterelement der Filtrierungsvorrichtung anschließend entnommen wird. Dies kann beispielsweise durch Abschrauben des Einlassabschnittes und/oder des Auslassabschnittes geschehen und dazu weisen die Filterkammer und der abzuschraubende Abschnitt vorzugsweise jeweils ein Gewinde auf, wie dies bereits obenstehend erläutert worden ist. Bevorzugt lässt sich der Auslassabschnitt auf diese Weise von der Filterkammer abschrauben. Dann kann das Filterelement einfach und funktionssicher aus der Filterkammer entfernt werden und weiterverwendet werden. Zudem kann nach dem Abschrauben bzw. Entfernen des Einlass- und/oder des Auslassabschnittes ein neues Filterelement in die Filterkammer eingebracht werden. Das im Rahmen der ersten Ausführungsform der Filterkammer entnommene, mit autologen Gewebebestandteilen versehene Filterelement ist vorzugsweise als ein mit autologen Gewebebestandteilen versehener Knochenersatzstoff, besonders bevorzugt als bioresorbierbarer Knochenersatzstoff, geeignet. Ebenso kann der im Rahmen der zweiten Ausführungsform dem Filterelement entnommene und mit autologen Gewebebestandteilen versehene Knochenersatzstoff analog eingesetzt werden.

Es empfiehlt sich, dass das mit den autologen Gewebebestandteilen versehene Filterelement bzw. der mit autologen Gewebebestandteilen versehene Knochenersatzstoff nach der Entnahme aus der Filtrierungsvorrichtung hinsichtlich seiner Form an den späteren Einsatzort bzw. Verwendungsort angepasst wird. Dazu kann im Rahmen der ersten Ausführungsform das Filterelement beispielsweise zerkleinert werden. Alternativ kann das Filterelement aber auch in seiner ursprünglichen Form weiterverwendet werden. Vorzugsweise verbleiben im Zuge der Anpassung die autologen Gewebebestandteile an dem Filterelement bzw. an dem Knochenersatzstoff.

Es liegt im Rahmen der Erfindung, dass es sich bei den autologen Gewebebestandteilen um Knochenfragmente und/oder Knochenmark und/oder Blutgerinnsel und/oder Fett handelt. Im Übrigen handelt es sich bei den autologen Gewebebestandteilen insbesondere um menschliche autologe Gewebebestandteile.

Gegenstand der Erfindung ist weiterhin eine Verwendung eines mit autologen Gewebebestandteilen versehenen Knochenersatzstoffes in Form eines Filterelementes und/oder in Form eines aus dem Filterelement entnommenen, mit autologen Gewebebestandteilen versehenen Knochenersatzstoffes, hergestellt durch ein obenstehend beschriebenes Verfahren, in einem Verfahren zur Behandlung von Knochendefekten im menschlichen Körper. Bei dem mit autologen Gewebebestandteilen versehenen Knochenersatzstoff handelt es sich bevorzugt um einen bioresorbierbaren synthetischen oder allogenen Knochenersatzstoff und besonders bevorzugt um ein Calciumphosphat, ganz besonders bevorzugt um β-Tricalciumphosphat und/oder um Hydroxylapatit. Wenn das mit autologen Gewebebestandteilen versehene Filterelement bzw. der mit autologen Gewebebestandteilen versehene Knochenersatzstoff aus der Filterkammer entnommen wird, kann es/er als Knochenersatzstoff in einem Verfahren zur Behandlung von Knochendefekten im menschlichen Körper eingesetzt werden. Dazu kann das Filterelement bzw. der Knochenersatzstoff hinsichtlich seiner Form an den Einsatzort bzw. Verwendungsort angepasst werden. Dieser Verwendung liegt die Erkenntnis zugrunde, dass ein bioresorbierbarer Knochenersatzstoff nach einer gewissen Zeit abgebaut ist und dass die autologen Gewebebestandteile ein osteoregeneratives Potential besitzen, also gleichsam die Knochenneubildung anregen. Auf diese Weise werden die während der Operation mit abgesaugten Gewebebestandteile, beispielsweise Knochenpartikel, nicht verworfen, sondern zusammen mit dem Filterelement aus einem bioresorbierbaren Knochenersatzstoff bzw. zusammen mit dem bioresorbierbaren Knochenersatzstoff zur Behandlung von Knochendefekten im menschlichen Körper verwendet. Dies ist sowohl wirtschaftlich als auch medizinisch äußerst vorteilhaft. Es empfiehlt sich, dass es sich bei dem verwendeten Knochenersatzstoff um β-Tricalciumphosphat handelt. Es kann auch Hydroxylapatit eingesetzt werden.

Der Erfindung liegt die Erkenntnis zugrunde, dass die erfindungsgemäße Filtrierungsvorrichtung einfach und wenig aufwendig handhabbar ist und dass zudem die Funktionssicherheit der Filtrierungsvorrichtung bzw. des Filterelementes über einen ausreichend langen Zeitraum gewährleistet ist. Wenn nach bevorzugter Ausführungsform der Erfindung der Einlassabschnitt und/oder der Auslassabschnitt reversibel von der Filterkammer entfernbar ist, zeichnet sich die Filtrierungsvorrichtung fernerhin dadurch aus, dass das Filterelement ausgetauscht werden kann und dass die Filterkammer und der Einlassabschnitt und der Auslassabschnitt wieder verwendet werden können. Ein ausgetauschtes und mit den autologen Gewebebestandteilen versehenes Filterelement kann dann als ein mit autologen Gewebebestandteilen versehener Knochenersatzstoff verwendet werden. Wenn das Filterelement gemäß der ersten Ausführungsform aus einem bioresorbierbaren Knochenersatzstoff besteht bzw. im Wesentlichen besteht, kann ein solches mit autologen Gewebebestandteilen versehenes Filterelement direkt als Knochenersatzstoff zur Behandlung von Knochendefekten im menschlichen Körper verwendet werden. Dies kann etwa im Rahmen derselben Operation geschehen, in der auch mit der chirurgischen Absaugvorrichtung ein Fluid abgesaugt wird bzw. in der mit der Filtrierungsvorrichtung autologe Gewebebestandteile abgetrennt werden. Insoweit zeichnet sich die erfindungsgemäße Filtrierungsvorrichtung dadurch aus, dass ihr Filterelement nach dem Filtrierungsvorgang nicht zusammen mit den aufgefangenen bzw. abgetrennten autologen Gewebebestandteilen verworfen werden muss, sondern dass das Filterelement bzw. der Knochenersatzstoff zur Behandlung von Knochendefekten eingesetzt werden kann. Die erfindungsgemäße Filtrierungsvorrichtung ist demzufolge äußerst funktionssicher, weist einen einfachen Aufbau auf, ermöglicht den problemlosen Austausch des Filterelementes und genügt in wirtschaftlicher Hinsicht allen Anforderungen. Es ist fernerhin anzumerken, dass die vorstehend beschriebenen Vorteile durch wenig aufwendige Maßnahmen erreicht werden können. Zudem macht die erfindungsgemäße Filtrierungsvorrichtung bzw. das erfindungsgemäße Verfahren zur Erzeugung eines mit autologen Gewebebestandteilen versehenen Knochenersatzstoffes es möglich, das osteoregenerative Potenzial von während einer Operation abgesaugten Gewebebestandteilen optimal auszunutzen. Die autologen Gewebebestandteile mit osteoregenerativem Potenzial müssen aufgrund der erfindungsgemäßen Maßnahmen nach der Absaugung bzw. Filtrierung weder verworfen werden, noch müssen sie in aufwendiger Weise von einem Filterelement abgetrennt werden. Insoweit zeichnet sich die Erfindung durch beachtliche Vorteile im Vergleich zu den aus der Praxis bekannten Maßnahmen aus.

Nachfolgend wird die Erfindung anhand einer lediglich ein Ausführungsbeispiel darstellenden Zeichnung näher erläutert. Es zeigen in schematischer Darstellung:
- Fig. 1: eine perspektivische Darstellung einer erfindungsgemäßen Filtrierungsvorrichtung,
- Fig. 2: eine Explosionsdarstellung des Gegenstandes nach Figur 1,
- Fig. 3: eine perspektivische Darstellung eines erfindungsgemäßen Filterelementes.

Die Erfindung betrifft eine Filtrierungsvorrichtung **1** für ein chirurgisches Absaugsystem. Die Filtrierungsvorrichtung **1** weist erfindungsgemäß einen Einlassabschnitt **2,** einen Auslassabschnitt **3** und eine Filterkammer **4** auf, wobei die Filterkammer **4** zwischen dem Einlassabschnitt **2** und dem Auslassabschnitt **3** angeordnet ist. In der Filterkammer **4** ist zweckmäßigerweise und im Ausführungsbeispiel ein Filterelement **5** angeordnet. Gemäß der ersten Ausführungsform der Erfindung ist das Filterelement **5** zumindest bereichsweise auf Basis zumindest eines Knochenersatzstoffes ausgebildet. Im Rahmen dieser Ausführungsform und im Ausführungsbeispiel besteht das Filterelement **5** aus einem Knochenersatzstoff und vorzugsweise aus einem bioresorbierbaren Knochenersatzstoff. Mittels des Filterelementes **5** sind autologe Gewebebestandteile, die in einem die Filtrierungsvorrichtung **1** durchströmenden Fluid mitgeführt werden, abtrennbar.

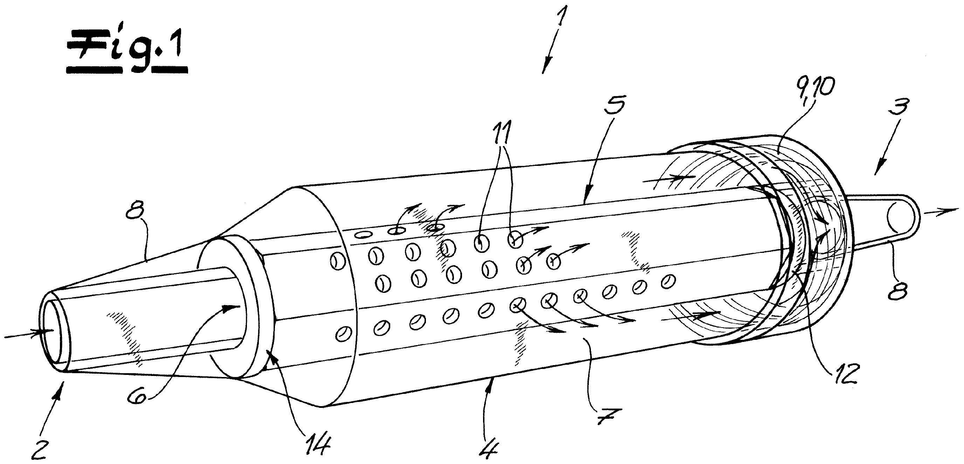

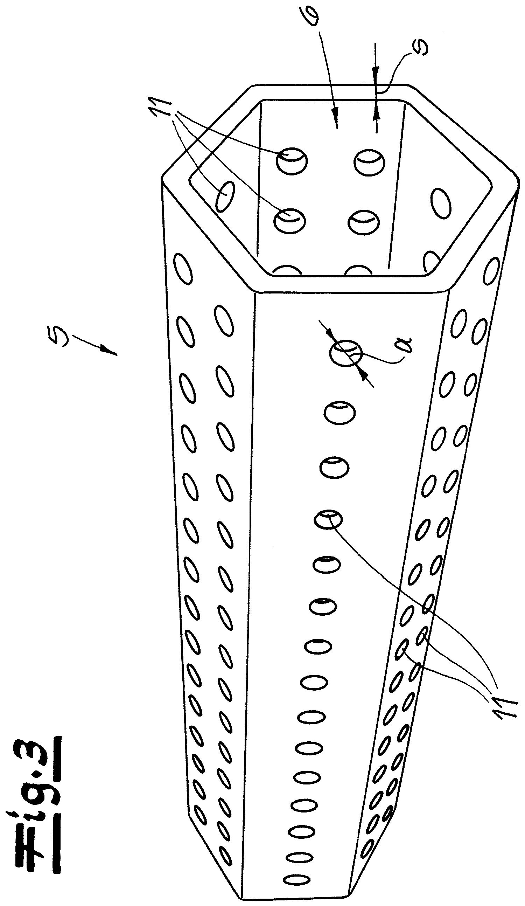

Bevorzugt ist das Filterelement als Hohlkörper ausgebildet und ganz besonders bevorzugt und im Ausführungsbeispiel gemäß den Figuren ist das Filterelement **5** röhrenförmig ausgebildet. Ein solches röhrenförmiges Filterelement **5** weist zweckmäßigerweise und im Ausführungsbeispiel jeweils an seinen Stirnseiten eine Öffnung auf. Das röhrenförmige Filterelement **5** weist zudem zweckmäßigerweise und im Ausführungsbeispiel einen inneren Strömungsraum **6** auf (siehe insbesondere Fig. 3). Empfohlenermaßen umgibt die Filterkammer **4** das Filterelement **5** (Fig. 1). Im Rahmen der Erfindung und im Ausführungsbeispiel umgibt die Filterkammer **4** das Filterelement **5** derart, dass zwischen der Außenseite des Filterelementes **5** und der Innenseite der Filterkammer **4** ein äußerer Strömungsraum **7** angeordnet ist. Dies zeigt insbesondere die Fig. 1.

Nach bevorzugter Ausführungsform und im Ausführungsbeispiel steht der innere Strömungsraum **6** in Fluidverbindung mit dem Einlassabschnitt **2** und der äußere Strömungsraum **7** steht in Fluidverbindung mit dem Auslassabschnitt **3.** Empfohlenermaßen und im Ausführungsbeispiel muss das die Filtrierungsvorrichtung **1** durchströmende Fluid das Filterelement **5** durchtreten, um von dem inneren Strömungsraum **6** in den äußeren Strömungsraum **7** zu gelangen. Vorzugsweise und im Ausführungsbeispiel steht dazu der Einlassabschnitt **2** filterkammerseitig ausschließlich in Fluidverbindung mit dem inneren Strömungsraum **6** und der Auslassabschnitt **3** steht filterkammerseitig ausschließlich in Fluidverbindung mit dem äußeren Strömungsraum **7.** Ausschließliche Fluidverbindung meint in diesem Zusammenhang die direkte Fluidverbindung, die nicht den Durchtritt durch das Filterelement meint bzw. ausschließt. Der resultierende Strömungsweg des Fluides ist in der Fig. 1 schematisch mit Pfeilen angedeutet.

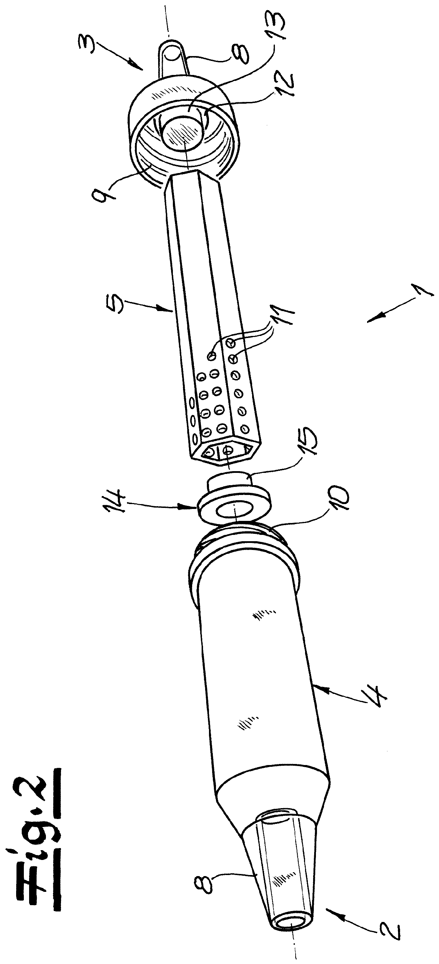

Zweckmäßigerweise ist das Filterelement **5** röhrenförmig ausgebildet und weist besonders bevorzugt und im Ausführungsbeispiel eine hexagonale Querschnittsfläche auf. Wenn nach empfohlener Ausführungsform und im Ausführungsbeispiel der Auslassabschnitt **3** filterkammerseitig ausschließlich in Fluidverbindung mit dem äußeren Strömungsraum **7** steht und der Einlassabschnitt **2** filterkammerseitig ausschließlich in Fluidverbindung mit dem inneren Strömungsraum **6** steht, dann ist zweckmäßigerweise und im Ausführungsbeispiel im montierten Zustand des Filterelementes **5** ein Ende des Filterelementes **5** - besonders bevorzugt das dem Auslassabschnitt **3** zugeordnete Ende des Filterelementes **5** - verschlossen. Montierter Zustand des Filterelementes **5** meint in diesem Zusammenhang insbesondere denjenigen Zustand des Filterelementes **5,** in dem sich das Filterelement **5** in der Filterkammer **4** befindet und in dem die Filtrierungsvorrichtung **1** einsatzbereit ist. Dieser Zustand ist in der Fig. 1 dargestellt. Zum Verschluss des einen Endes des röhrenförmigen Filterelementes **5** im montierten Zustand ist vorzugsweise ein Verschlusselement, bevorzugt und im Ausführungsbeispiel eine Verschlussplatte **12** an dem Auslassabschnitt **3** angeordnet, die im montierten Zustand des Filterelementes **5** das dem Auslassabschnitt **3** zugeordnete Ende des Filterelementes **5** verschließt. Vorzugsweise und im Ausführungsbeispiel weist die Verschlussplatte **12** einen Befestigungsfortsatz **13** auf (Fig. 2), der in das Filterelement **5** eingesteckt bzw. eingeschoben werden kann, sodass der Befestigungsfortsatz im montierten Zustand des Filterelementes **5** in das Filterelement **5** hineinreicht (Fig. 1).

Zweckmäßigerweise und im Ausführungsbeispiel gemäß den Figuren ist die Filterkammer **4** röhrenförmig ausgebildet. Empfohlenermaßen und im Ausführungsbeispiel sind sowohl das Filterelement **5,** als auch die Filterkammer **4** röhrenförmig ausgebildet und vorzugsweise umgibt die röhrenförmige Filterkammer **4** das röhrenförmige Filterelement **5.** Es empfiehlt sich, dass das Filterelement **5** und die Filterkammer **4** koaxial zueinander angeordnet sind (Fig. 1). Bevorzugt und im Ausführungsbeispiel erstreckt sich das Filterelement in seiner Längsausdehnung über die gesamte Länge der Filterkammer bzw. im Wesentlichen über die gesamte Länge der Filterkammer.

Zweckmäßigerweise und im Ausführungsbeispiel weisen der Einlassabschnitt **2** und der Auslassabschnitt **3** an der der Filterkammer **4** abgewandten Seite einen Anschlussstutzen **8** auf. Ein solcher Anschlussstutzen **8** dient zum Anschluss weiterer Elemente des chirurgischen Absaugsystems. Beispielsweise kann an dem Anschlussstutzen **8** des Auslassabschnittes **3** ein Schlauch angeschlossen werden, um das aus der Filtrierungsvorrichtung **1** herausströmende Fluid weiterzutransportieren. Der Anschlussstutzen **8** des Einlassabschnittes **2** kann beispielsweise dazu verwendet werden, ein Saugrohr bzw. einen Saugrüssel aufzunehmen.

An dem Einlassabschnitt **2** ist zweckmäßigerweise und im Ausführungsbeispiel ein Manschettenelement **14** mit einem Befestigungsfortsatz **15** vorgesehen, auf den das Filterelement **5** aufgesteckt bzw. aufgeschoben werden kann, sodass der Befestigungsfortsatz **15** im montierten Zustand des Filterelementes **5** in das Filterelement **5** hineinreicht. Vorzugsweise und im Ausführungsbeispiel wird das Filterelement **5** im montierten Zustand also von dem Befestigungsfortsatz **13** der Verschlussplatte **12** und dem Befestigungsfortsatz **15** des Manschettenelementes **14** in der Filterkammer **4** gehalten.

Gemäß einer bevorzugten Ausführungsform und im Ausführungsbeispiel ist an dem Auslassabschnitt **3** filterkammerseitig zumindest ein Verbindungselement in Form eines Innengewindes **9** angeordnet, das mit einem an der Filterkammer **4** angeordneten komplementären Verbindungselement in Form eines Komplementäraußengewindes **10** wechselwirkt. Auf diese Weise ist der Auslassabschnitt **3** reversibel von der Filterkammer **4** entfernbar, bevorzugt und im Ausführungsbeispiel reversibel abschraubbar. Dann kann das Filterelement **5** einfach und funktionssicher aus der Filterkammer **4** entnommen werden und durch ein neues Filterelement **5** ersetzt werden.

Das Innengewinde **9** des Auslassabschnittes **3** und das Komplementäraußengewinde **10** der Filterkammer **4** sind insbesondere in der Fig. 2 zu erkennen. Ihre Wechselwirkung bzw. ihr verschraubter Zustand ist insbesondere in der Fig. 1 dargestellt.

Vorzugsweise beträgt die Stärke bzw. Wandstärke s des Filterelementes **5** zwischen 0,7 mm und 3,0 mm, besonders bevorzugt zwischen 1,0 mm und 2,5 mm und ganz besonders bevorzugt zwischen 1,2 mm und 2,0 mm. Im Ausführungsbeispiel (siehe insbesondere Fig. 3) mag die Stärke bzw. Wandstärke s des Filterelementes **5** etwa 1,8 mm betragen. Stärke bzw. Wandstärke s des Filterelementes **5** meint in diesem Zusammenhang insbesondere die Dicke bzw. die Durchtrittsdicke des Filterelementes **5.**

Gemäß ganz besonders bevorzugter Ausführungsform und im Ausführungsbeispiel weist das Filterelement **5** eine Vielzahl von Fluiddurchtrittsöffnungen **11** auf. Zweckmäßigerweise und im Ausführungsbeispiel sind die Fluiddurchtrittsöffnungen **11** als Rundlöcher ausgebildet. Es empfiehlt sich, dass die Fluiddurchtrittsöffnungen **11** einen Durchmesser a im Bereich von 0,2 mm bis 6,0 mm, bevorzugt zwischen 0,5 mm und 4,0 mm und besonders bevorzugt zwischen 1,0 mm und 3,0 mm aufweisen. Im Ausführungsbeispiel (siehe insbesondere Fig. 3) mag der Durchmesser a der Fluiddurchtrittsöffnungen **11** etwa 2,5 mm betragen. Durchmesser a meint in diesem Zusammenhang insbesondere den größten Durchmesser der Fluiddurchtrittsöffnung **11,** also im Falle eines Rundloches den Kreisdurchmesser.

Es empfiehlt sich, dass der Flächenanteil der Fluiddurchtrittsöffnungen **11** in Bezug auf die Oberfläche des Filterelementes **5** zwischen 2 % und 20 %, bevorzugt zwischen 3 % und 15 % und ganz besonders bevorzugt zwischen 4% und 12 % beträgt. Im Ausführungsbeispiel gemäß der Fig. 3 mag der Flächenanteil der Fluiddurchtrittsöffnungen **11** in Bezug auf die Oberfläche des Filterelementes **5** etwa 9 % betragen. Vorzugsweise und im Ausführungsbeispiel (Fig. 3) sind die Fluiddurchtrittsöffnungen **11** gleichmäßig bzw. im Wesentlichen gleichmäßig entlang des Filterelementes **5** bzw. der Oberfläche des Filterelementes **5** angeordnet. Empfohlenermaßen und im Ausführungsbeispiel sind die Fluiddurchtrittsöffnungen **11** in Längsrichtung des Filterelementes **5** mit konstantem Abstand bzw. im Wesentlichen mit konstantem Abstand zueinander angeordnet.

Zweckmäßigerweise und im Ausführungsbeispiel kann das mit dem chirurgischen Absaugsystem abgesaugte Fluid durch den Einlassabschnitt **2,** der vorzugsweise und im Ausführungsbeispiel filterkammerseitig ausschließlich in Fluidverbindung mit dem inneren Strömungsraum **6** des röhrenförmigen Filterelementes **5** steht, in den inneren Strömungsraum **6** einströmen. Zur Erzeugung der Saugwirkung weist das chirurgische Absaugsystem üblicherweise eine Pumpe oder dergleichen auf. Im montierten Zustand des Filterelementes **5,** der in der Fig. 1 dargestellt ist, ist vorzugsweise und im Ausführungsbeispiel zumindest ein Ende des Filterelementes verschlossen ausgebildet. Im Ausführungsbeispiel (s. Fig. 1) ist das dem Auslassabschnitt **3** zugewandte bzw. zugeordnete Stirnende des röhrenförmigen Filterelementes **5** durch die Verschlussplatte **12** verschlossen, die an dem Auslassabschnitt **3** angeordnet ist und im montierten Zustand des Filterelementes **5** ein Ende des Filterelementes **5** verschließt. Zweckmäßigerweise und im Ausführungsbeispiel muss dann das die Filtrierungsvorrichtung **1** durchströmende Fluid das Filterelement **5** durchtreten, um von dem inneren Strömungsraum **6** in den äußeren Strömungsraum **7** zu gelangen. Zum Durchtritt des Filterelementes **5** weist das Filterelement **5** vorzugsweise und im Ausführungsbeispiel eine Vielzahl von Fluiddurchtrittsöffnungen **11** auf. Nach dem Durchtritt des Fluides durch die Fluiddurchtrittsöffnungen **11** verbleibt zumindest ein Teil der autologen Gewebebestandteile, die in dem Fluid mitgeführt wurden aufgrund ihrer Größe in dem Filterelement **5.** Das in dem äußeren Strömungsraum **7** befindliche Fluid kann die Filtrierungsvorrichtung dann durch den Auslassabschnitt **3,** der vorzugsweise und im Ausführungsbeispiel filterkammerseitig ausschließlich in Fluidverbindung mit dem äußeren Strömungsraum **7** steht, verlassen. Die ausschließliche Fluidverbindung meint in diesem Zusammenhang im Übrigen nicht die Fluidverbindung der Strömungsräume aufgrund der Fluiddurchtrittsöffnungen **11** des Filterelementes **5.**

Bei der ersten Ausführungsform der Erfindung besteht das Filterelement **5** aus dem zumindest einen Knochenersatzstoff bzw. besteht das Filterelement **5** im Wesentlichen aus dem zumindest einen Knochenersatzstoff. Es empfiehlt sich, dass es sich bei dem Knochenersatzstoff um einen bioresorbierbaren Knochenersatzstoff, vorzugsweise um ein Calciumsalz einer Säure ausgewählt aus der Gruppe: "Phosphorsäure, Schwefelsäure, Kohlensäure" handelt. Empfohlenermaßen und im Ausführungsbeispiel handelt es sich bei dem Knochenersatzstoff bzw. bioresorbierbaren Knochenersatzstoff um β-Tricalciumphosphat.

Es liegt im Rahmen der Erfindung, dass die Filterkammer **4** und/oder der Einlassabschnitt **2** und/oder der Auslassabschnitt **3** aus zumindest einem polymeren Kunststoff bestehen. Vorzugsweise und im Ausführungsbeispiel besteht das Aggregat aus Filterkammer **4** und Einlassabschnitt **2** und Auslassabschnitt **3** aus PMMA bzw. im Wesentlichen aus PMMA.

## Patentansprüche

1. Filtrierungsvorrichtung für ein chirurgisches Absaugsystem, wobei die Filtrierungsvorrichtung (1) einen Einlassabschnitt (2), einen Auslassabschnitt (3) und eine Filterkammer (4) aufweist, wobei die Filterkammer (4) zwischen dem Einlassabschnitt (2) und dem Auslassabschnitt (3) angeordnet ist, wobei in der Filterkammer (4) zumindest ein Filterelement (5) angeordnet ist, wobei
**entweder** das Filterelement (5) zumindest bereichsweise auf Basis zumindest eines Knochenersatzstoffes - vorzugsweise eines bioresorbierbaren Knochenersatzstoffes-ausgebildet ist
**oder** das Filterelement (5) zumindest bereichsweise auf Basis zumindest eines Trägermaterials - insbesondere auf Basis zumindest eines Kunststoffes - ausgebildet ist, wobei innerhalb des Filterelementes (5) zumindest ein Knochenersatzstoff - vorzugsweise zumindest ein bioresorbierbarer Knochenersatzstoff - angeordnet ist
und wobei mittels des Filterelementes (5) autologe Gewebebestandteile, die in einem die Filtrierungsvorrichtung (1) durchströmenden Fluid mitgeführt werden, abtrennbar sind.

2. Filtrierungsvorrichtung nach Anspruch 1, wobei das Trägermaterial bzw. der Kunststoff ein polymerer Kunststoff, vorzugsweise ein polymeres Acrylat oder ein polymeres Methacrylat ist und insbesondere Polymethylmethacrylat (PMMA) ist.

3. Filtrierungsvorrichtung nach einem der Ansprüche 1 oder 2, wobei der Knochenersatzstoff - insbesondere der bioresorbierbare Knochenersatzstoff - in Form von Teilchen bzw. kleinen Teilchen und insbesondere in Form von Granulatteilchen innerhalb des Filterelementes (5) angeordnet ist.

4. Filtrierungsvorrichtung nach einem der Ansprüche 1 bis 3, wobei das Filterelement (5) als Hohlkörper ausgebildet ist und einen inneren Strömungsraum (6) aufweist, wobei die Filterkammer (4) das Filterelement (5) umgibt und vorzugsweise derart umgibt, dass zwischen der Außenseite des Filterelementes (5) und der Innenseite der Filterkammer (4) ein äußerer Strömungsraum (7) angeordnet ist.

5. Filtrierungsvorrichtung nach Anspruch 4, wobei der innere Strömungsraum (6) in Fluidverbindung mit dem Einlassabschnitt (2) steht, wobei der äußere Strömungsraum (7) in Fluidverbindung mit dem Auslassabschnitt (3) steht und wobei vorzugsweise das die Filtrierungsvorrichtung (1) durchströmende Fluid das Filterelement (5) durchtreten muss, um von dem inneren Strömungsraum (6) in den äußeren Strömungsraum (7) zu gelangen.

6. Filtrierungsvorrichtung nach einem der Ansprüche 4 oder 5, wobei der Einlassabschnitt (2) filterkammerseitig ausschließlich in Fluidverbindung mit dem inneren Strömungsraum (6) steht und/oder wobei der Auslassabschnitt (3) filterkammerseitig ausschließlich in Fluidverbindung mit dem äußeren Strömungsraum (7) steht.

7. Filtrierungsvorrichtung nach einem der Ansprüche 1 bis 6, wobei das Filterelement (5) röhrenförmig ausgebildet ist und wobei im montierten Zustand des Filterelementes (5) vorzugsweise zumindest ein Ende des Filterelementes (5) verschlossen ist.

8. Filtrierungsvorrichtung nach einem der Ansprüche 1 bis 7, wobei das Filterelement (5) und die Filterkammer (4) koaxial zueinander angeordnet sind.

9. Filtrierungsvorrichtung nach einem der Ansprüche 1 bis 8, wobei die Stärke bzw. Wandstärke s des Filterelementes (5) zwischen 0,5 mm und 4,0 mm bevorzugt zwischen 0,7 mm und 3,0 mm, besonders bevorzugt zwischen 1,0 mm und 2,5 mm und ganz besonders bevorzugt zwischen 1,2 mm und 2,0 mm beträgt.

10. Filtrierungsvorrichtung nach einem der Ansprüche 1 bis 9, wobei das Filterelement (5) eine Vielzahl von Fluiddurchtrittsöffnungen (11) aufweist, wobei die Fluiddurchtrittsöffnungen (11) einen Durchmesser a im Bereich von 0,1 mm bis 8,0 mm, bevorzugt zwischen 0,2 mm und 6,0 mm, besonders bevorzugt zwischen 0,5 mm und 4,0 mm und ganz besonders bevorzugt zwischen 1,0 mm und 3,0 mm aufweisen.

11. Filtrierungsvorrichtung nach einem der Ansprüche 1 bis 10, wobei es sich bei dem Knochenersatzstoff um einen bioresorbierbaren Knochenersatzstoff, vorzugsweise um ein Calciumsalz einer Säure ausgewählt aus der Gruppe: "Phosphorsäure, Schwefelsäure, Kohlensäure", besonders bevorzugt um ein Calciumphosphat und ganz besonders bevorzugt um β-Tricalciumphosphat handelt.

12. Verfahren zur Erzeugung eines mit autologen Gewebebestandteilen versehenen Knochenersatzstoffes, wobei in eine Filtrierungsvorrichtung für ein chirurgisches Absaugsystem nach einem der Ansprüche 1 bis 11 über einen Einlassabschnitt (2) ein Fluid, das autologe Gewebebestandteile enthält, eingesaugt wird, wobei das Fluid anschließend in eine Filterkammer (4) strömt, in der zumindest ein Filterelement (5) angeordnet ist, wobei
**entweder** das Filterelement (5) zumindest bereichsweise auf Basis zumindest eines Knochenersatzstoffes - vorzugsweise eines bioresorbierbaren Knochenersatzstoffes - ausgebildet ist
**oder** das Filterelement (5) zumindest bereichsweise auf Basis zumindest eines Trägermaterials - insbesondere auf Basis zumindest eines Kunststoffes - ausgebildet ist, wobei innerhalb des Filterelementes (5) zumindest ein Knochenersatzstoff - vorzugsweise ein bioresorbierbarer Knochenersatzstoff - angeordnet ist,
wobei innerhalb des Filterelementes (5) zumindest ein Knochenersatzstoff - vorzugsweise ein bioresorbierbarer Knochenersatzstoff - angeordnet ist, wobei das Fluid das Filterelement (5) durchtritt, wobei zumindest ein Teil der autologen Gewebebestandteile von dem Filterelement (5) bzw. von dem Knochenersatzstoff aufgefangen bzw. zurückgehalten wird und wobei das filtrierte Fluid anschließend durch einen Auslassabschnitt (3) aus der Filtrierungsvorrichtung (1) herausströmt.

13. Verfahren nach Anspruch 12, wobei das mit den autologen Gewebebestandteilen versehene Filterelement (5) bzw. der mit den autologen Gewebebestandteilen versehene Knochenersatzstoff der Filtrierungsvorrichtung (1) entnommen wird und insbesondere zur weiteren Verwendung als ein mit autologen Gewebebestandteilen versehener Knochenersatzstoff geeignet ist.

14. Verfahren nach einem der Ansprüche 12 oder 13, wobei es sich bei den autologen Gewebebestandteilen um Knochenfragmente und/oder Knochenmark und/oder Blutgerinnsel und/oder Fett handelt.

## Claims

1. Filter assembly for a surgical suction system, wherein the filter assembly (1) has an intake fitting (2), an output fitting (3) and a filter chamber (4), wherein the filter chamber (4) is arranged between the intake fitting (2) and the output fitting (3), wherein at least one filter element (5) is arranged inside the filter chamber (4), wherein
**either** at least a region of the filter element (5) is constructed on the basis of at least one bone substitute material - preferably a bioresorbable bone substitute material
**or** at least a region of the filter element (5) is constructed on the basis of at least one support material - particularly on the basis of at least one plastic -, wherein at least one bone substitute material - preferably at least one bioresorbable bone substitute material - is arranged inside the filter element (5),
and wherein autologous tissue components which are entrained in a fluid flowing through the filter assembly (1) can be separated by the filter element (5) .

2. Filter assembly according to Claim 1, wherein the support material or plastic is a polymeric plastic, preferably a polymeric acrylate or a polymeric methacrylate, and in particular is polymethyl methacrylate (PMMA).

3. Filter assembly according to either Claim 1 or 2, wherein the bone substitute material - particularly the bioresorbable bone substitute material - is arranged inside the filter element (5) in the form of particles and/or small particles, and in particular in the form of granules.

4. Filter assembly according to any one of Claims 1 to 3, wherein the filter element (5) is constructed as a hollow body and has an inner flow cavity (6), wherein the filter chamber (4) surrounds the filter element (5), and preferably surrounds it in such manner that an outer flow cavity (7) is formed between the outer side of the filter element (5) the inner side of the filter chamber (4).

5. Filter assembly according to Claim 4, wherein the inner flow cavity (6) is in fluid connection with the inlet fitting (2), wherein the outer flow cavity (7) is in fluid connection with the outlet fitting (3), and wherein preferably the fluid flowing through the filter assembly (1) must pass through the filter element (5) in order to enter the outer flow cavity (7) from the inner flow cavity (6).

6. Filter assembly according to either of Claims 4 or 5, wherein the inlet fitting (2) is in fluid connection solely with the inner flow cavity (6) on the filter chamber side and/or wherein the outlet fitting (3) is in fluid connection solely with the outer flow cavity (7) on the filter chamber side.

7. Filter assembly according to any one of Claims 1 to 6, wherein the filter element (5) is of tubular construction, and wherein preferably at least one end of the filter element (5) is blocked when the filter element (5) is in the assembled state.

8. Filter assembly according to any one of Claims 1 to 7, wherein the filter element (5) and the filter chamber (4) are arranged coaxially with each other.

9. Filter assembly according to any one of Claims 1 to 8, wherein the thickness or wall thickness s of the filter element (5) has a value between 0.5 mm and 4.0 mm, preferably between 0.7 mm and 3.0 mm, particularly preferably between 1.0 mm and 2.5 mm, and most particularly preferably between 1.2 mm and 2.0 mm.

10. Filter assembly according to any one of Claims 1 to 9, wherein the filter element (5) has a plurality of fluid passages (11), wherein the fluid passages (11) have a diameter a in the range from 0.1 mm to 8.0 mm, preferably between 0.2 mm and 6.0 mm, particularly preferably between 0.5 mm and 4.0 mm, and most particularly preferably between 1.0 mm and 3.0 mm.

11. Filter assembly according to any one of Claims 1 to 10, wherein the bone substitute material is a bioresorbable bone substitute material, preferably a calcium salt of an acid selected from the group: "phosphoric acid, sulfuric acid, carbonic acid", particularly preferably a calcium phosphate most particularly preferably β-tricalcium phosphate.

12. Method for producing a bone substitute material which includes autologous tissue components, wherein a fluid containing autologous tissue components is sucked through an inlet fitting (2) into a filter assembly for a surgical suction system according to any of Claims 1 to 11, wherein the fluid then flows into a filter chamber (4) in which at least one filter element (5) is arranged, wherein
**either** at least a region of the filter element (5) is constructed on the basis of at least one bone substitute material - preferably a bioresorbable bone substitute material
**or** at least a region of the filter element (5) is constructed on the basis of at least one support material - particularly on the basis of at least one plastic -, wherein at least one bone substitute material - preferably a bioresorbable bone substitute material - is arranged inside the filter element (5), wherein at least one bone substitute material - preferably a bioresorbable bone substitute material - is arranged inside the filter element (5), wherein the fluid passes through the filter element (5), wherein at least a part of the autologous tissue components is collected and trapped by the filter element (5) or the bone substitute material, and wherein the filtered fluid then flows out of the filter assembly (1) through an outlet fitting (3).

13. Method according to Claim 12, wherein filter element (5) having the autologous tissue components or the bone substitute material of the filter assembly (1) having the autologous tissue components is removed from the filter assembly (1) and in particular is suitable for further use as a bone substitute material which has a content of autologous tissue components.

14. Method according to either of Claims 12 or 13, wherein the autologous tissue components are bone fragments and/or bone marrow and/or blood clots and/or fat.

## Revendications

1. Dispositif de filtration pour un système d'aspiration chirurgical, dans lequel le dispositif de filtration (1) présente un tronçon d'admission (2), un tronçon d'évacuation (3) et une chambre de filtre (4), dans lequel la chambre de filtre (4) est disposée entre la section d'admission (2) et la section d'évacuation (3), dans lequel au moins un élément de filtre (5) est disposé dans la chambre de filtre (4), dans lequel
**soit** l'élément de filtre (5) est composé au moins par tronçons à base d'au moins un matériau de substitut osseux, de préférence un matériau de substitut osseux bio-résorbable
**soit** l'élément de filtre (5) est composé au moins par tronçons à base d'au moins un matériau porteur, en particulier à base d'au moins un plastique, dans lequel au moins un matériau de substitut osseux, de préférence au moins un matériau de substitut osseux bio-résorbable, est disposé à l'intérieur de l'élément de filtre (5)
et dans lequel des composants tissulaires autologues qui sont entraînés dans un fluide traversant le dispositif de filtration (1), peuvent être séparées au moyen de l'élément de filtration (5).

2. Dispositif de filtration selon la revendication 1, dans lequel le matériau porteur, respectivement le plastique, est un plastique polymère, de préférence un acrylate polymère ou un méthacrylate polymère et en particulier du polyméthyle méthacrylate (PMMA).

3. Dispositif de filtration selon l'une des revendications 1 ou 2, dans lequel le matériau de substitut osseux, de préférence le matériau de substitut osseux bio-résorbable, est disposé sous forme de particules, respectivement de petites particules et en particulier sous forme de particules de granulat à l'intérieur de l'élément de filtre (5).

4. Dispositif de filtration selon l'une des revendications 1 à 3, dans lequel l'élément de filtre (5) est conçu en tant que corps creux et présente un espace d'écoulement intérieur (6), dans lequel la chambre de filtre (4) entoure l'élément de filtre (5) et l'entoure de préférence de telle façon qu'un espace d'écoulement extérieur (7) est disposé entre la face extérieure de l'élément de filtre (5) et la face intérieure de la chambre de filtre (4).

5. Dispositif de filtration selon la revendication 4, dans lequel l'espace d'écoulement intérieur (6) est en liaison fluidique avec le tronçon d'admission (2), dans lequel l'espace d'écoulement extérieur (7) est en liaison fluidique avec le tronçon d'évacuation (3) et dans lequel de préférence, le fluide traversant le dispositif de filtration (1) doit traverser l'élément de filtre (5) pour parvenir de l'espace d'écoulement intérieur (6) à l'espace d'écoulement extérieur (7).

6. Dispositif de filtration selon l'une des revendications 4 ou 5, dans lequel le tronçon d'admission (2) est exclusivement en liaison fluidique avec l'espace d'écoulement intérieur (6) côté chambre de filtre et/ou dans lequel le tronçon d'évacuation (3) est exclusivement en liaison fluidique avec l'espace d'écoulement extérieur (7) côté chambre de filtre.

7. Dispositif de filtration selon l'une des revendications 1 à 6, dans lequel l'élément de filtre (5) est conçu tubulaire et dans lequel, dans l'état monté de l'élément de filtre (5), de préférence au moins une extrémité de l'élément de filtre (5) est fermée.

8. Dispositif de filtration selon l'une des revendications 1 à 7, dans lequel l'élément de filtre (5) et la chambre de filtre (4) sont disposés coaxialement l'un à l'autre.

9. Dispositif de filtration selon l'une des revendications 1 à 8, dans lequel l'épaisseur, respectivement l'épaisseur de paroi s de l'élément de filtre (5) fait entre 0,5 mm et 4,0 mm, de préférence entre 0,7 mm et 3,0 mm, particulièrement de préférence entre 1,0 mm et 2,5 mm et tout particulièrement de préférence entre 1,2 mm et 2,0 mm.

10. Dispositif de filtration selon l'une des revendications 1 à 9, dans lequel l'élément de filtre (5) présente une pluralité d'ouvertures de passage de fluide (11), dans lequel les ouvertures de passage de fluide (11) présentent un diamètre a dans la plage de 0,1 mm à 8,0 mm, de préférence entre 0,2 mm et 6,0 mm, particulièrement de préférence entre 0,5 mm et 4,0 mm et tout particulièrement de préférence entre 1,0 mm et 3,0 mm.

11. Dispositif de filtration selon l'une des revendications 1 à 10, dans lequel le matériau de substitut osseux est un matériau de substitut osseux bio-résorbable, de préférence un sel de calcium d'un acide sélectionné parmi le groupe : « acide phosphorique, acide sulfurique, acide carbonique », particulièrement de préférence un phosphate de calcium et tout particulièrement de préférence un phosphate de tri-calcium β.

12. Procédé destiné à la fabrication d'un matériau de substitut osseux doté de composants tissulaires autologues, dans lequel dans un dispositif de filtration pour un système d'aspiration chirurgical selon l'une des revendications 1 à 11, un fluide qui contient des composants tissulaires autologues est aspiré par le biais d'un tronçon d'admission (2), dans lequel le fluide s'écoule ensuite dans une chambre de filtre (4) dans laquelle au moins un élément de filtre (5) est disposé, dans lequel
**soit** l'élément de filtre (5) est composé au moins par tronçons à base d'au moins un matériau de substitut osseux, de préférence un matériau de substitut osseux bio-résorbable
**soit** l'élément de filtre (5) est composé au moins par tronçons à base d'au moins un matériau porteur, en particulier à base d'au moins un plastique, dans lequel au moins un matériau de substitut osseux, de préférence un matériau de substitut osseux bio-résorbable, est disposé à l'intérieur de l'élément de filtre (5)
dans lequel au moins un matériau de substitut osseux, de préférence un matériau de substitut osseux bio-résorbable, est disposé à l'intérieur de l'élément de filtre (5), dans lequel le fluide traverse l'élément de filtre (5), dans lequel au moins une partie des composants tissulaires autologues est piégée respectivement retenue par l'élément de filtre (5), respectivement par le matériau de substitut osseux et dans lequel le fluide filtré sort ensuite du dispositif de filtration (1) par un tronçon d'évacuation (3).

13. Procédé selon la revendication 12, dans lequel l'élément de filtre (5) doté des composants tissulaires autologues, respectivement le matériau de substitut osseux doté des composants tissulaires autologues, est prélevé du dispositif de filtration (1) et convient en particulier pour une application ultérieure en tant que matériau de substitut osseux doté de composants tissulaires autologues.

14. Procédé selon la revendication 12 ou 13, dans lequel les composants tissulaires autologues sont des fragments osseux et/ou de la moelle osseuse et/ou des caillots et/ou de la graisse.
